(19) **Europäisches Patentamt**

**European Patent Office**

**Office européen des brevets**

(11) Publication number: **0 402 553 B1**

## EUROPEAN PATENT SPECIFICATION

(45) Date of publication of patent specification: **21.09.94**  (51) Int. Cl.⁵: **A61M 5/145**, F16H 25/24

(21) Application number: **89600024.7**

(22) Date of filing: **27.11.89**

(54) **Syringe pump.**

(30) Priority: **15.06.89 GR 89010400**

(43) Date of publication of application:
**19.12.90 Bulletin 90/51**

(45) Publication of the grant of the patent:
**21.09.94 Bulletin 94/38**

(84) Designated Contracting States:
**DE FR GB**

(56) References cited:
**EP-A- 0 204 977**        **DE-A- 1 750 562**
**FR-A- 2 518 410**        **GB-A- 2 166 497**
**US-A- 4 563 175**        **US-A- 4 838 857**

(73) Proprietor: **MICREL LTD, MICROELECTRONIC APPLICATIONS CENTER**
**30 Amiklon and Kefalinias Street**
**GR-152 31 Halandri, Athens (GR)**

(72) Inventor: **Tsoukalis, Alexandre**
**Amyclon 30**
**GR-15231 K. Chalandri Athens (GR)**

(74) Representative: **Eisenführ, Speiser & Partner**
**Martinistrasse 24**
**D-28195 Bremen (DE)**

## Description

The invention relates to a syringe infusion pump comprising a lead screw translator for translating rotational movement of a drive motor into linear movement of a carriage guided in parallel to the axis of the lead screw and acting on a plunger of a syringe, the carriage being disengageable from the lead screw for permitting exchange of a syringe. Such an infusion pump is already known from US-A-4 838 857.

In modern therapy, several liquids need to be infused into the body at a controlled rate. Several types of infusion pumps have been developed, one of them named syringe infusion pump consisting of a mechanism for pushing a syringe's plunger and administering liquid via a catheter to a body.

Such a pump must fulfil the following recommendations especially if it is to be used also for parenteral (or intravenous) administration: Be precise and safe, be rudged and wear resistant, be easy to use, control the pressure of infused liquid under precise low and high limits, be adjustable for rates and for syringes of different brands by a safe and error-free mechanism. It is the aim of the present invention to provide improvements in several technological solutions concerned by these recommendations.

A syringe pump, as for instance known from US-A-4 838 857, using an externally screw-threaded drive rod as driving principles (also called a lead screw translator), has to provide the facility to push the syringe's plunger during normal operation, but also to let the pushing member be placed freely behind the plunger by an engage/disengage/engage procedure of the driving mechanism and the drive rod, the known mechanism fails to fulfil the wear resistant recommendation. When the syringe has a back force due to venal or arterial pressure or to an occlusion, a disengagement of the pushing member for changing the syringe, disengages the driving half nuts which receive a tangential force that may damage the half nuts as they move out of engagement with the drive rod, passing over several threads of the lead screw before they are at last disengaged. The same damage is encountered if one engages the mechanism while moving the pushing member, the only coupling mechanism being the nuts themselves. At least one proposal exists that uses speedy disengagement as a remedy, which lessens but does not eliminate the problem.

From US-A-4 563 175 a spindle drive for the plunger of a syringe is known, in which a nut is permanently coupled with the lead screw of the spindle drive. The coupling between the driving gear and the spindle is provided by a releasable friction clutch.

Moreover, GB-A-2 166 497 discloses an infusion pump in which the plunger of a syringe is actuated by a mechanical energy store, i.e. a spring. The spring is loaded or primed by hand operation assisted by a ratchet/pawl mechanism permitting rotation of a worm wheel in one direction but locking rotation in the other direction. The worm wheel engages a worm gear which can be rotated by a battery-operated electric motor to controllably retard and release the energy stored in the spring to the plunger.

It is an object of the present invention to provide a syringe infusion pump comprising a lead screw translator, in which the wear of the mechanism is drastically reduced and the reliability of the driving system is improved.

According to the present invention, a syringe infusion pump as defined above is characterized by at least one worm wheel rotatably supported on the carriage and permanently engaging the external thread of the lead screw driven by the drive motor, and an actuating mechanism controlling a brake for normally arresting rotation of the worm wheel in both directions for transmission of linear movement, and for releasing the worm wheel for free rotation in both directions and disengaging the transmission of linear movement.

One embodiment of the invention is characterized by in addition to said worm wheel, a lever pivotally mounted on the carriage and having a half nut normally engaging the lead screw, and the actuating mechanism controlling the brake for the worm wheel as well as operating the lever for moving the half nut out of engagement with the lead screw.

An alternative embodiment is characterized by two worm wheels rotatably supported on the carriage on opposite sides of the lead screw, the brake normally arresting the worm wheels and being adapted for releasing the worm wheels at the same time.

Further embodiments are defined in the dependent claims.

According to the syringe infusion pump of the present invention, including the various embodiments, the reliability of the driving system is improved. By using a brake the half nut is engaged or disengaged only in a relatively stationary system without being damaged, as the three functions half nut engage, brake, lock, are done by a precise sequence in each engage/disengage/engage procedure. The brake is used in conjunction with the property of a crown wheel (worm wheel) of a worm gear to roll over an externally screw-threaded drive rod (lead screw), and the movement is transmitted during braking operation to the worm gear working as a half nut.

As a syringe pump preferably has to signal an alarm for high limit of pressure inside the syringe's barrel, and also for low pressure which indicates a misplacement of the canula, a sensor has to be placed at some location. The direct method of measuring at the fluid needs a special type of catheter as a disposable with a soft membrane part, which is not economical against the standard one. Another known method is to sense the pressure through a strain gaugge placed near the motor, thus sensing through the total system friction from the syringe's plunger to the motor, passing by at least three friction parts. In the known solutions, using smaller than the calibrated syringe in diameter can make the pump exceed the high pressure limits by a large extent. By the present invention, the sensing element is placed right on the pushing member, just in touch with the syringe's plunger. No pump's friction member is placed in between. An analog to digital converter gives a direct reaction force measurement and pressure is calculated by the system microprocessor by the relation $P = C1*F/S + C2$, where P stands for pressure, C1 and C2 are calibration factors for each syringe type, F for A/D reading, S for used syringe's cross sectional area. It is apparent that by the present invention any syringe type has the same safe pressure limits.

As a syringe pump preferably has to be able to adapt for different syringes, a safe label-digital input set for each syringe has to be used so that accidental use of different setting be eliminated. In the known pumps, a BCD or Hexadecimal switch is used which can be readjusted from one nurse to the next, leading to errors of use. In the present invention, a special type of plug having redundant (for safety) brigded leads selectively for each syringe type, is used as digital input information to the processor and having a large, colored in different background-letter combinations labelling area, so that safety is improved by making any change pass through an authorized person who holds the connectors and by clearly labelling each change.

The invention will now be described by way of examples in connection with the accompanying drawings, in which

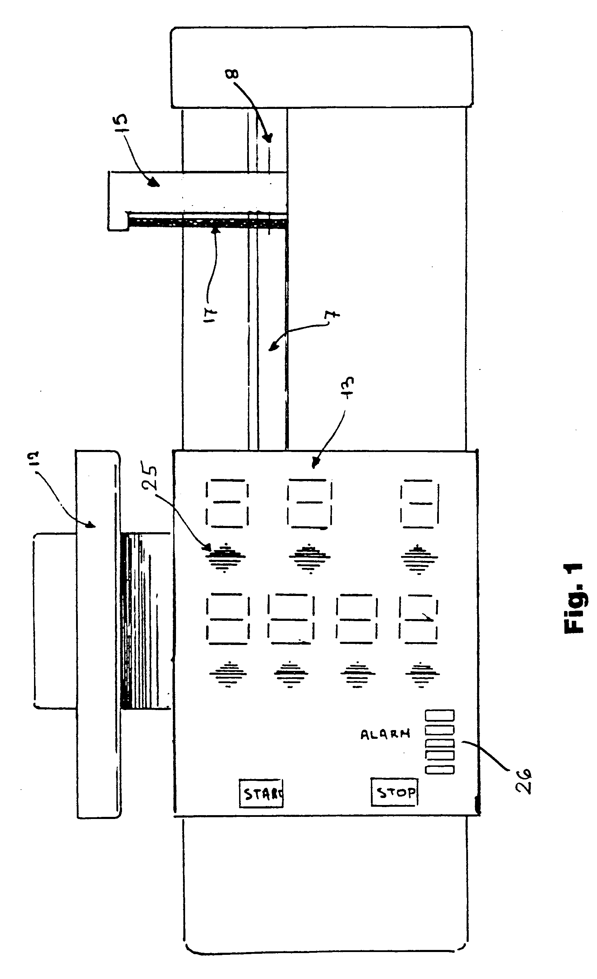

Figure 1    shows the outer appearance of the syringe infusion pump;

Figure 2    shows a plug selector for the syringe type;

Figure 3    shows details of a mechanism with a worm wheel and a half nut as well as a brake and a lock driving mechanism;

Figure 4    shows a side view of the syringe pump; and

Figure 5    shows another embodiment with two worm wheels and a modified brake mechanism.

The preferred embodiment consists of a casing having a multiple keyboard 25 and multiple displays 13, alarm indicators 26, cover for syringe retention 12, inside the casing a motor 18 drivably connected with an externally threaded lead screw 1. The rotation of the motor 18 is translated to linear movement via a half nut 5 and a worm wheel 2 to a carriage and moving rods 7 and 11, which slide over a guide rod 8. Rod 7 is fixed at one end to a pushing member 17, at the outer end fixed to the carriage 9 retaining the worm wheel 2 which is adjacent to the wall of the casing in a way that it cannot rotate around the guide rod 8 which means that member 17 does not rotate either. On member 17 and in touch with the syringe's plunger is placed a linear force sensor assembly 16. Rod 11 is at one end fixed to a member 15 which can be rotated by hand in a way that it holds or releases the end of the syringe's plunger, passing over member 17. At the other end, rod 11 is fixed to a cam 14, which rotates together with member 15. A lever 10 is pivotally supported by a pivot 23 on carriage 9, and both parts form a lock mechanism over lead screw 1, a spring 6 tending to close the lock mechanism around lead screw 1, stopped by their form in a way that no vertical force is exercised on lead screw 1, being just in close tolerance (with clearance) engaged with it. A lock pin 4 (engaging a bore 4a) is used for said lock mechanism not to open if excessive force is exercised in the lead screw. Spring 6 is not of critical torque value, and it can be made weaker than if its function were to hold parts 10 and 9 in driving engagement with lead screw 1. A brake 3 is used to stop worm wheel 2 rotating, transforming it to a half nut in function, so that an engagement or disengagement of the main driving half nut 5 is done in a relative stationary system, i.e. regardless if the motor is running or not, as the threads of half nut 5 and worm wheel 2 are synchronized by holes 21 along worm wheel's side where brake (arresting pin) 3 enters, each hole 21 being a stable location for each discrete possible match of threads of half nut 5 and lead screw 1. With this system it is apparent that no damage of main half nut 5 is possible, because brake 3 itself has low damage as it is designed for such a braking function. Worm wheel 2 is of nonstandard thread, having many deep threads engaged with the lead screw 1, designed to hold by itself alone the relatively high loads that a hand can exert on member 15. This system improves life expectancy of the infusion pump, and as electronics are very reliable, the weakest part of the previous designs were the driving half nuts, clipped out by the engagement-disengagement procedure. If cam 14 is rotated through moving rod 11 and member 15 by hand, it enduces the following sequence of functions in the disengagement procedure:

    a) Full engaged system with lock 4 in, brake 3 in, half nut 5 engaged, translating rotation to linear movement.

    b) Lock 4 out.

    c) Half nut 5 disengaged.

    d) Brake 3 out, free linear travel.

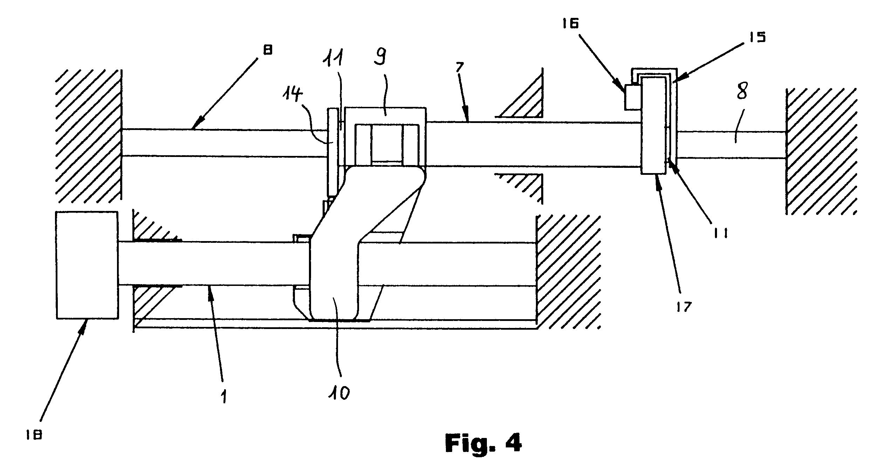

The inverse is done for the engagement procedure as illustrated in Figure 3.

Cam 14 when completely rotated for free run, is blocked by a protrusion 24 of lever 10 so that it finds a second stable position.

In Figure 5, another implementation of the same braking principle as in conjunction with the worm wheel 2 of Figure 3 is illustrated, instead two worm wheels 2a, 2b are on each side of the lead screw 1, roll over it at the disengagement position and are braked by arresting pins 3a, 3b through rotation of the cam 14 at engagement, thus being transformed functionally to half nuts.

In the present invention there are no sliding parts (when at run) over the casing, but only two journal bearings at the ends of rods 7 and 11 with guide rod 8 and their penetrating the casing journal bearing, inducing the minimal and smoothest friction along the travel, which means minimal oscillations if a self adapting P.I.D. control and a DC motor is used.

A signal of the strain gauge 16 can be transferred through rods 7 and 11, who form a coaxial like cable, down to the carriage 9 which at its side can have a magnetic or optic sensor for absolute position reading in accordance with an adequate strip along the casing. Any type of switch can be used for sensing if parts 9 and 10 are locked. Singals of switch, encoder and force sensor are transferred to a safe system of microprocessors through a flexible cable. Motor 8 may have fixed on its rotor a magnetic drum and hall effect sensors that are used as tachogenerators. Its current is limited, so that a fault in the pressure transducer will not make the pump exceed the maximum allowed pressure limits.

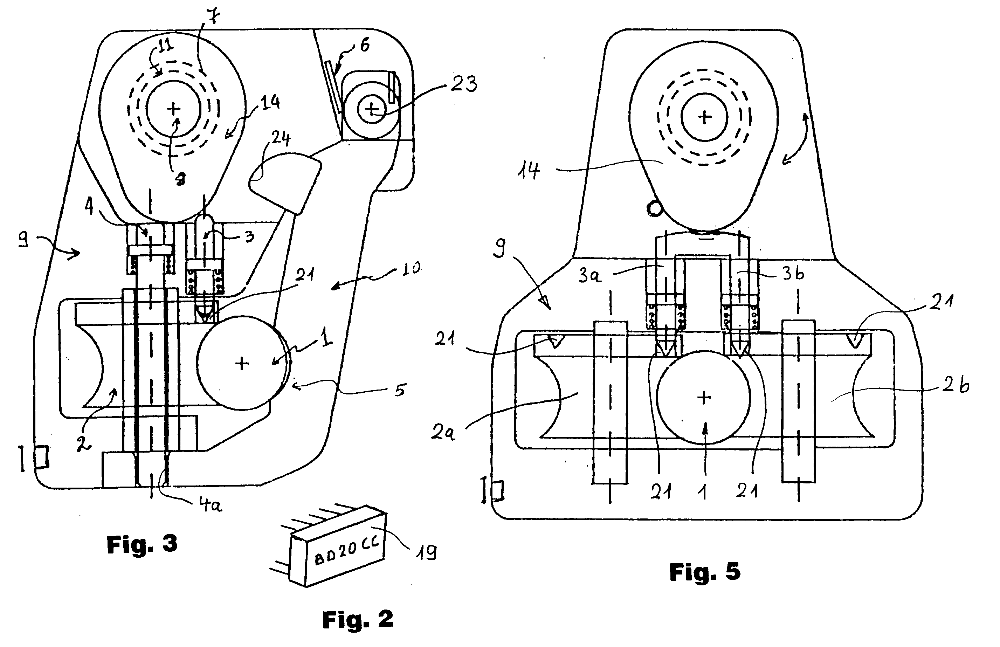

In Figure 2 the plug type syringe selector 19 is illustrated, where a bicolored label signals the used syringe's brand name and volume, having different sets of colors for the different combinations of syringes, and where the plug's pins are internally selectively bridged for giving a digital information on the syringe used to the microprocessor. Two redundant sets of said bridged pins are used for safety.

## Claims

1. A syringe infusion pump comprising a lead screw translator for translating rotational movement of a drive motor (18) into linear movement of a carriage (9) guided in parallel to the axis of the lead screw (1) and acting on a plunger of a syringe, the carriage (9) being disengageable from the lead screw (1) for permitting exchange of a syringe,
characterized by
at least one worm wheel (2; 2a, 2b) rotatably supported on the carriage (9) and permanently engaging the external thread of the lead screw (1) driven by the drive motor (18), and
an actuating mechanism (11, 14) controlling a brake (3; 3a, 3b) for normally arresting rotation of the worm wheel (2; 2a, 2b) in both directions for transmission of linear movement, and for releasing the worm wheel (2; 2a, 2b) for free rotation in both directions and disengaging the transmission of linear movement.

2. A syringe infusion pump according to claim 1, characterized by, in addition to said worm wheel (2), a lever (10) pivotally mounted on the carriage (9) and having a half nut (5) normally engaging the lead screw (1), and the actuating mechanism (11, 14) controlling the brake (3) for the worm wheel (2) as well as operating the lever (10) for moving the half nut (5) out of engagement with the lead screw (1).

3. A syringe infusion pump according to claim 1, characterized by two worm wheels (2a, 2b) rotatably supported on the carriage (9) on opposite sides of the lead screw (1), the brake (3) normally arresting the worm wheels (2a, 2b) and being adapted for releasing the worm wheels (2a, 2b) at the same time.

4. A syringe infusion pump according to any of claims 1 to 3,
characterized by a member (15) operated by hand action for holding the plunger of the syringe and also operating the actuating mechanism (11, 14).

5. A syringe infusion pump according to claim 2, characterized by a locking mechanism (4, 4a) between the carriage (9) and the lever (10) and being controlled by the actuating mechanism (11, 14) for normally locking the half nut (5) in the engaged position with the lead screw (1).

6. A syringe infusion pump according to any preceding claim,
characterized in that the brake (3) is an arresting pin engaging apertures (21) around each of said worm wheels (2; 2a, 2b).

7. A syringe infusion pump according to claims 2 and 6,
characterized in that the positions of the apertures (21) are matched to the threads of lead screw (1).

8. A syringe infusion pump according to any preceding claim,
characterized in that the actuating mechanism (11, 14) is a cam mechanism.

9. A syringe infusion pump according to claims 2, 5 and 6,
characterized in that the cam mechanism (11, 14) is adapted to provide the following phases for disengaging the transmission:
a) releasing the locking mechanism (11, 14) between the carriage (9) and the lever (10),
b) operating the lever (10) for moving the half nut (5) out of engagement with the lead screw (1),
c) releasing the brake (3) from the worm wheel (2).

**Patentansprüche**

1. Spritzeninfusionspumpe mit einem Schraubenspindelgetriebe zum Umwandeln einer Drehbewegung eines Antriebsmotors (18) in eine Linearbewegung eines Schlittens (9), der parallel zur Achse der Schraubenspindel (1) geführt ist und auf einen Kolben einer Spritze einwirkt, wobei der Schlitten (9) von der Schraubenspindel (1) zum Austausch der Spritze auskuppelbar ist,
gekennzeichnet durch
mindestens ein Scheckenrad (2; 2a, 2b), das drehbar auf dem Schlitten (9) gelagert ist und in die äußeren Schneckengänge der durch den Antriebsmotor (18) angetriebenen Schraubenspindel (1) eingreift; und einen Betätigungsmechanismus (11, 14), der eine Bremse (3; 3a, 3b) derart steuert, daß diese normalerweise zur Erzielung einer Linearbewegung ein Drehen des Schneckenrades (2; 2a, 2b) in beiden Richtungen verhindert und zur Unterbrechung der Übertragung der Linearbewegung das Schneckenrad (2; 2a, 2b) zur freien Drehung in beiden Richtungen freigibt.

2. Spritzeninfusionspumpe nach Anspruch 1,
dadurch gekennzeichnet, daß zusätzlich zu dem Schneckenrad (2) ein Hebel (10) schwenkbar an dem Schlitten (9) gelagert sowie eine Halbmutter (5) vorgesehen ist, die normalerweise in die Schraubenspindel (1) eingreift, und daß der Betätigungsmechanismus (11, 14) sowohl die Bremse (3) des Schneckenrades (3) als auch den Hebel (10) betätigt, um die Halbmutter (5) außer Eingriff mit der Schraubenspindel (1) zu bringen.

3. Spritzeninfusionspumpe nach Anspruch 1,
dadurch gekennzeichnet, daß zwei Schneckenräder (2a, 2b) drehbar an dem Schlitten (a) auf einander gegenüberliegenden Seiten der Schraubenspindel (1) gelagert sind, und daß die Bremse (3) normalerweise die Schneckenräder (2a, 2b) arretiert und dazu ausgebildet ist, die Schneckenräder gleichzeitig freizugeben.

4. Spritzeninfusionspumpe nach einem der Ansprüche 1-3,
gekennzeichnet durch ein durch handbetätigtes Glied (15) zum Halten des Kolbens der Spritze und zum Aktivieren des Betätigungsmechanismus (11, 14).

5. Spritzeninfusionspumpe nach Anspruch 2,
gekennzeichnet durch einen zwischen dem Schlitten (9) und dem Hebel (10) angeordneten Verriegelungsmechanismus (4, 4a), der durch den Betätigungsmechanismus (11, 14) derart betätigbar ist, daß er normalerweise die Halbmutter (5) im Eingriffszustand mit der Schraubenspindel (1) verriegelt.

6. Spritzeninfusionspumpe nach einem der vorstehenden Ansprüche,
dadurch gekennzeichnet, daß die Bremse (3) als Arretierstift ausgebildet ist, der in Öffnungen (21) eingreift, die um jedes der Schneckenräder (2; 2a, 2b) angeordnet sind.

7. Spritzeninfusionspumpe nach den Ansprüchen 2 und 6,
dadurch gekennzeichnet, daß die Positionen der Öffnungen (21) an die Schneckengänge der Schraubenspindel (1) angepaßt sind.

8. Spritzeninfusionspumpe nach einem der vorstehenden Ansprüche,
dadurch gekennzeichnet, daß der Betätigungsmechanismus (11, 14) ein Nockenmechanismus ist.

9. Spritzeninfusionspumpe nach den Ansprüchen 2, 5 und 6,
dadurch gekennzeichnet, daß der Nockenmechanismus (11, 14) dazu ausgebildet ist, die folgenden Phasen beim Trennen der Bewegungsübertragung durchzuführen:

a) Freigeben des Verriegelungsmechanismus (11, 14) zwischen dem Schlitten (9)
und dem Hebel (10),
b) Betätigen des Hebels (9) zum Bewegen
der Halbmutter (5) außer Eingriff mit der
Schraubenspindel (1),
c) Freigeben der Bremse (3) aus dem
Schneckenrad (2).

**Revendications**

1. Pousse-seringue de perfusion comprenant un dispositif de transformation de mouvement à vis-mère pour transformer le mouvement rotatif d'un moteur d'entraînement (18) en déplacement linéaire d'un chariot (9) guidé parallèlement à l'axe de la vis-mère (1) et agissant sur le piston d'une seringue, le chariot (9) pouvant être débrayé de la vis-mère (1) pour permettre le changement de seringue caractérisée par au moins une roue sans fin (2; 2a, 2b) montée en rotation sur le chariot (9) et s'engrenant de manière permanente sur le filetage extérieur de la vis-mère (1) entraînée par le moteur d'entraînement (18), et un mécanisme d'actionnement (11, 14) qui commande un frein (3; 3a, 3b) pour arrêter normalement la rotation de la roue sans fin (2; 2a, 2b) dans l'une ou l'autre direction de transmission du mouvement linéaire, et pour libérer la roue sans fin (2; 2a, 2b) de façon qu'elle puisse tourner en roue libre dans l'une ou l'autre direction et interrompre la transmission du mouvement linéaire.

2. Pousse-seringue de perfusion selon la revendication 1, caractérisée, en plus de la roue sans fin (2), par un levier (10) monté en pivot sur le chariot (9) et comportant un demi-écrou (5) qui s'engrène normalement sur la vis-mère (1), le mécanisme d'actionnement (11, 14) commandant le frein (3) de la roue sans fin (2) ainsi que le levier de manoeuvre (10) permettant de désengrener le demi-écrou (5) de la vis-mère (1).

3. Pousse-seringue de perfusion selon la revendication 1, caractérisée par deux roues sans fin (2a, 2b) montées en rotation autour d'axes logés sur le chariot (9), situées respectivement chacune à une des extrémités de la vis-mère (1), et par le frein (3) arrêtant normalement les roues sans fin (2a, 2b) et étant prévu pour libérer ces roues sans fin (2a, 2b) simultanément.

4. Pousse-seringue de perfusion selon l'une quelconque des revendications 1 à 3, caractérisée par un élément (15) actionné à la main pour maintenir le piston de la seringue, ainsi que pour manoeuvrer le mécanisme d'actionnement (11, 14).

5. Pousse-seringue de perfusion selon la revendication 2, caractérisée par un mécanisme de blocage (4, 4a) entre le chariot (9) et le levier (10) et commandé par le mécanisme d'actionnement (11, 14) pour bloquer normalement le demi-écrou (5) en position engrenée sur la vis-mère (1).

6. Pousse-seringue de perfusion selon l'une quelconque des revendications précédentes, caractérisée en ce que le frein (3) est une cheville d'arrêt qui s'engage dans des ouvertures (21) à la périphérie des roues sans fin (2; 2a, 2b).

7. Pousse-seringue de perfusion selon les revendications 2 et 6 caractérisée en ce que les positions des ouvertures (21) sont en alignement avec les filets de la vis-mère (1).

8. Pousse-seringue de perfusion selon l'une quelconque des revendications précédentes, caractérisée en ce que le mécanisme d'actionnement (11, 14) est un mécanisme à came.

9. Pousse-seringue de perfusion selon les revendications 2, 5 et 6, caractérisée en ce que le mécanisme à came (11, 14) est étudié pour exécuter les phases suivantes pour déverrouiller la transmission:
(a) déblocage du mécanisme de blocage (11, 14) entre le chariot (9) et le levier (10),
(b) manoeuvre du levier (10) pour désengrener le demi-écrou (5) de la vis-mère (1),
(c) libération du frein (3) de la vis-mère (2).

**Fig. 1**

EP 0 402 553 B1

**Fig. 3**

**Fig. 2**

**Fig. 5**

EP 0 402 553 B1

**Fig. 4**